# EUROPEAN PATENT APPLICATION

(11) **EP 4 442 120 A1**
(43) Date of publication of application: **09.10.2024**
(21) Application number: 22900345.4
(22) Date of filing: 23.11.2022
(51) Int. Cl.: A23C 9/123, A23C 17/02, A23C 9/16, A23L 2/39, A23L 2/52, A23K 10/18, A61K 35/745, A61P 39/06, A61P 37/04, C12N 1/20

(54) **NEW USE OF BIFIDOBACTERIUM INFANTIS YLGB-1496 IN ANTI-AGING AND IMPROVEMENT OF INNATE IMMUNITY**

(30) Priority: 30.11.2021 CN 202111453508
(71) Applicant: Inner Mongolia Yili Industrial Group Co., Ltd., Inner Mongolia 010000 (CN); Inner Mongolia Dairy Tech Res Institute Co Ltd, Hohhot, Inner Mongolia 010110 (CN)
(72) Inventor: LIU, Wei-Hsien, Hohhot, Inner Mongolia 010000 (CN); ZHAO, Wen, Hohhot, Inner Mongolia 010000 (CN); HUNG, Wei-Lian, Hohhot, Inner Mongolia 010000 (CN); WEISS, Gisela Adrienne, Hohhot, Inner Mongolia 010000 (CN); VAN LOO-BOUWMAN, Carolien Annika, Hohhot, Inner Mongolia 010000 (CN)
(74) Representative: Dehns
(86) International application number: PCT/CN2022/133810
(87) International publication number: WO 2023/098540

(57) **Abstract**

Disclosed is the use of a *Bifidobacterium longum* subsp. *infantis* strain in the preparation of a composition for anti-aging in individuals, improving innate immunity in individuals, relieving symptoms caused by *Staphylococcus aureus* infections in individuals and/or inhibiting *Staphylococcus aureus* infection in individuals, wherein the *Bifidobacterium infantis* is *Bifidobacterium infantis* with the deposit number of CGMCC NO. 21109. A composition for anti-aging in individuals, improving innate immunity in individuals, relieving symptoms caused by *Staphylococcus aureus* infections in individuals and/or inhibiting *Staphylococcus aureus* infection in individuals.

## Description

### Technical Field

The present invention relates to the technical field of microorganisms, and in particular to the new use of a *Bifidobacterium longum* subsp. *infantis* (i.e., *Bifidobacterium infantis*) strain YLGB-1496 (with the deposit number of CGMCC No. 21109) in alleviating death caused by *Staphylococcus aureus* infections, prolonging lifespan, improving innate immunity and delaying aging.

### Background Art

Of the various physiological processes in the body, the immune system is the most vulnerable to attack. The immune system is in constant contact with most organs and cells in the body, and its changes are bound to affect the tissue cells of those organs. With increasing age, the function of the immune system gradually decreases, and both the innate immune response and the acquired immune response decrease accordingly, causing some diseases that seriously affect tissues and organs and aggravating the aging of various systems of the body. Immunosenescence refers to a phenomenon of susceptibility to chronic and acute diseases due to dysregulation of the immune function. The decline of the immune function is one of the important factors causing the aging of the body, and therefore strengthening the body's immune function can reduce the occurrence of pathological aging.

Probiotics are single or well-defined mixtures of microorganisms that produce beneficial effects on host health by changing the composition of the flora in a specific part of the host. The probiotic effect refers to a beneficial physiological effect of a specific microbial preparation or fermented product on the host by regulating the host's mucosal and systemic immune functions and improving intestinal nutrition and flora balance. As the aging population increases, functional foods that can provide probiotic effects, control aging, and prolong lifespan are becoming more popular. HIROMI KIMOTO-NIRA et al. used SAMP6 in the senescence accelerated mouse (SAM) model and found that SAMP6 mice treated with heat-inactivated *Lactococcus lactis* subsp. *cremoris* H61, produced more IL-12 and IFN-y in splenocytes. This study suggests that the ability of *Lactococcus lactis* subsp. *cremoris* H61 to inhibit certain aging phenomena may be related to its immunomodulatory effects.

*Caenorhabditis elegans* (C. *elegans*) is a common, free-living, small soil nematode, belonging to the subclass *Rhabditia,* the order *Rhabditidia,* the superfamily *Rhabditoidea. C. elegans* feeds on bacteria and has a life cycle of approximately 3 days, with an average lifespan of 3 weeks. It is worm-like, with an adult length of about 1.0-1.5 mm and a body diameter of about 70.0 µm. *C. elegans* is bilaterally symmetrical, with a layer of cuticle covering the body surface, no segmentation, 4 main epidermal cord-like tissues, and 1 pseudocoelom filled with body fluid. The biological characteristics of C. *elegans,* such as its simple structure, transparent body, ease of observation, and short reproductive cycle, provide the C. *elegans* with the premise of becoming a model organism. Studying the mechanisms of aging and lifespan control in nematodes and applying the results to other organisms are of great significance for prolonging human lifespan and improving human quality of life. The experiment explores the anti-aging effect of probiotics in the body by observing the lifespan of the model animal C. *elegans* and provides a theoretical reference for the development and utilization of probiotic products with anti-aging and innate immunity-improving effects.

### Summary of the Invention

An objective of the present invention is to provide the new use of *Bifidobacterium longum* subsp. *infantis* YLGB-1496.

The *Bifidobacterium longum* subsp. *infantis* YLGB-1496 has the deposit number of CGMCC No. 21109. The *Bifidobacterium longum* subsp. *infantis* strain with the deposit number of CGMCC No. 21109 was deposited in China General Microbiological Culture Collection Center (CGMCC) on December 05, 2020, with the address of depositary institution: Institute of Microbiology, Chinese Academy of Sciences, No. 3, Yard 1, Beichen West Road, Chaoyang District, Beijing; date of deposit: December 05, 2020; deposit number: CGMCC No. 21109; classified nomenclature: *Bifidobacterium longum* subsp. *infantis.* In the present invention, this strain is also called YLGB-1496 or GB-1496.

The present invention has found that the *Bifidobacterium longum* subsp. *infantis* strain YLGB-1496 has the effects of alleviating death caused by *Staphylococcus aureus* infections, prolonging lifespan and improving innate immunity.

Therefore, the present invention provides the use of *Bifidobacterium longum* subsp. *infantis* in the preparation of a composition for use in anti-aging in individuals, improving innate immunity in individuals, relieving symptoms caused by *Staphylococcus aureus* infections in individuals and/or inhibiting *Staphylococcus aureus* infection in individuals, wherein the strain is *Bifidobacterium longum* subsp. *infantis* with the deposit number of CGMCC No. 21109.

According to a specific embodiment of the present invention, the symptoms caused by *Staphylococcus aureus* infections in the present invention include aging (e.g., immunosenescence) and even death.

In another aspect, the present invention further provides a composition for use in anti-aging in individuals, improving innate immunity in individuals, relieving symptoms caused by *Staphylococcus aureus* infections in individuals and/or inhibiting *Staphylococcus aureus* infection in individuals, which composition comprises an effective amount of *Bifidobacterium longum* subsp. *infantis* with the deposit number of CGMCC No. 21109.

According to a specific embodiment of the present invention, the *Bifidobacterium infantis* is used in the form of a solid or liquid bacterial preparation for the preparation of the composition.

According to a specific embodiment of the present invention, the composition may include a food composition, a feed composition or a pharmaceutical composition.

According to a specific embodiment of the present invention, the composition may be used in animals or humans. The composition may further comprise conventional material components in the art. For example, the pharmaceutical composition may comprise an appropriate amount of auxiliary material, and the auxiliary material may be an excipient, a diluent, a filler and/or an absorption enhancer, etc. The food composition containing the *Bifidobacterium infantis* of the present invention can be produced in accordance with the food containing *Bifidobacterium infantis* in the prior art. Depending on the needs of the recipient, the composition may be in various forms, such as a powder, a lozenge, a granulate, a microcapsule and/or a liquid preparation.

According to a specific embodiment of the present invention, the composition is used to alleviate death caused by *Staphylococcus aureus* infections; in the specific use, the *Bifidobacterium infantis* is used in an amount of 1.0 × 10³ CFU-1.0 × 10¹² CFU/day, preferably 1.0 × 10⁷ CFU-1.0 × 10¹⁰ CFU/day.

According to a specific embodiment of the present invention, the composition is used to prolong lifespan; in the specific use, the *Bifidobacterium infantis* is used in an amount of 1.0 × 10³ CFU-1.0 × 10¹² CFU/day, preferably 1.0 × 10⁷ CFU-1.0 × 10¹⁰ CFU/day.

According to a specific embodiment of the present invention, the composition is used to improve innate immunity; in the specific use, the *Bifidobacterium infantis* is used in an amount of 1.0 × 10³ CFU-1.0 × 10¹² CFU/day, preferably 1.0 × 10⁷ CFU-1.0 × 10¹⁰ CFU/day.

In a specific embodiment of the present invention, the composition is a food composition, wherein the food is a fermented dairy product (such as fermented dairy, flavored fermented dairy, and fermented dairy beverage), a cheese, a dairy-containing beverage, a solid beverage, or a dairy powder (such as infant formula dairy powder or non-infant formula dairy powder).

In another aspect, the present invention further provides a method for anti-aging, improving innate immunity in individuals, relieving symptoms caused by *Staphylococcus aureus* infections in individuals and/or inhibiting *Staphylococcus aureus* infection in individuals, which method comprises administering to an individual in need thereof an effective amount of *Bifidobacterium longum* subsp. *infantis* with the deposit number of CGMCC No. 21109.

In some specific embodiments of the present invention, the anti-aging is anti-immunosenescence.

According to a specific embodiment of the present invention, the *Bifidobacterium longum* subsp. *infantis* is administered to the individual in an amount of 1.0 × 10³ CFU-1.0 × 10¹² CFU/day, preferably 1.0 × 10⁷ CFU-1.0 × 10¹⁰ CFU/day.

In conclusion, the present invention provides the new use of *Bifidobacterium infantis* YLGB-1496, wherein the strain has the effects of remarkably alleviating death caused by *Staphylococcus aureus* infections, prolonging lifespan, and improving innate immunity, can be used to prepare foods, medicines, feeds and the like with anti-aging and innate immunity-improving effects, and has wide application prospects.

### Brief Description of the Drawings

FIG. 1 shows the pathogenicity of S. *aureus* to C. *elegans* and the effect of adding a probiotic strain.
FIG. 2 shows the survival rate of C. *elegans* after 5 days of S. *aureus* and probiotic intervention.

### Deposit of Microorganisms for the Purposes of Patent Procedure

*Bifidobacterium infantis* strain YLGB-1496 (or GB-1496):
Date of deposit: December 05, 2020;
Depositary institution: China General Microbiological Culture Collection Center (CGMCC);
Address of depositary institution: Institute of Microbiology, Chinese Academy of Sciences, No. 3, Yard 1, Beichen West Road, Chaoyang District, Beijing;
Deposit number: CGMCC No. 21109;
Classified nomenclature: *Bifidobacterium longum* subsp. *infantis.*

### Detailed Description of Embodiments

In order to understand the technical features, objectives and beneficial effects of the present invention more clearly, the technical solutions of the present invention are described in detail below in conjunction with specific examples. It should be understood that these examples are only used to illustrate the present invention rather than limit the scope of the present invention. In the examples, the reagent raw materials are commercially available, and experimental methods without specifying specific conditions are conventional methods and conditions well known in the art, or are conducted according to conditions suggested by an instrument manufacturer.

### Example 1: Test of anti-aging effect of probiotics on C. elegans

### 1.1 Materials and instruments

*Bifidobacterium infantis* (i.e., *Bifidobacterium longum* subsp. *infantis)* YLGB-1496 is available from Inner Mongolia Yili Industrial Group Co., Ltd. (sourced from breast milk of healthy Chinese mothers, deposited in China General Microbiological Culture Collection Center (CGMCC) on December 05, 2020, with the address of depositary institution: Institute of Microbiology, Chinese Academy of Sciences, No. 3, Yard 1, Beichen West Road, Chaoyang District, Beijing; date of deposit: December 05, 2020; deposit number: CGMCC No. 21109; classified nomenclature: *Bifidobacterium longum* subsp. *infantis*).

*Bifidobacterium lactis* commercial strain X.

Wild-type *Caenorhabditis elegans* (*C*. *elegans*) is commercially available.

*Staphylococcus aureus* (ATCC25923) is commercially available.

Other reagents used in the experiment are purchased from Sinopharm Chemical Reagent Co., Ltd. SPX-150B-Z biochemical incubator: Shanghai Boxun Industrial Co., Ltd. Medical Equipment Factory; Olympus microscope (OlympusBX41): Nanjing Ailang Instrument Co., Ltd.; LDZX-50KBS Vertical Pressure Steam Sterilizer: Shanghai Shen'an Medical Appliance Factory; T6 ultraviolet/visible spectrophotometer: Beijing Puxi General Instrument Co., Ltd.; Multiskan FC microplate reader: Thermo Fisher Scientific (Shanghai) Instruments Co., Ltd.

### 1.2 Experimental method

### 1.2.1 Culture method for C. elegans

An NGM medium was used. Preparation method: 1000 mL of culture medium containing 17 g of agar, 2.5 g of polypeptone, and 3 g of NaCl was sterilized by high-pressure steam at 121°C for 20 min; 1 mL of 1 mol/L CaCl₂, 1 mL of 1 mol/L MgSO₄, and 25 mL of 1 mol/L K₃PO₄ buffer solution after sterilization were added; when the mixture was cooled to about 50°C, 1 mL of 5 mg/mL cholesterol was added, shaken uniformly, and poured into a sterile culture dish; OP50 was placed in the culture dish and cultured overnight at 37°C for later use.

### 12.2 Activation of probiotics

*Lactobacillus* was cultured in an MRS medium and *Bifidobacterium* was cultured in an MRS+Cys (1%) medium at 37°C for activation; the cells were collected, eluted with normal saline to adjust to 1 × 10⁷ and 1 × 10⁸ CFU (the amount of probiotics in each culture dish), and placed in an NGM medium for later use.

### 1.2.3 Determination of C. elegans lifespan

The exposed *C*. *elegans* were randomly picked and placed in the NGM medium coated with *Bifidobacterium lactis* (commercial strain X), *Lactobacillus paracasei* K56, and *Bifidobacterium infantis* YLGB-1496. The NGM medium containing *Escherichia coli* OP50 and a culture medium without other components (only *S*. *aureus*) were used as control groups. Each group was set with 3 replicates and incubated at 20°C. The number of survival days of *C*. *elegans* was counted from the day of transfer (day 0 of the survival experiment); the *C*. *elegans* was considered dead if it remained motionless when touched by a platinum wire; the number of living and dead *C. elegans* was recorded daily; the experiment lasted until day 5, and the mortality was recorded.

### 1.2.4 Experimental results

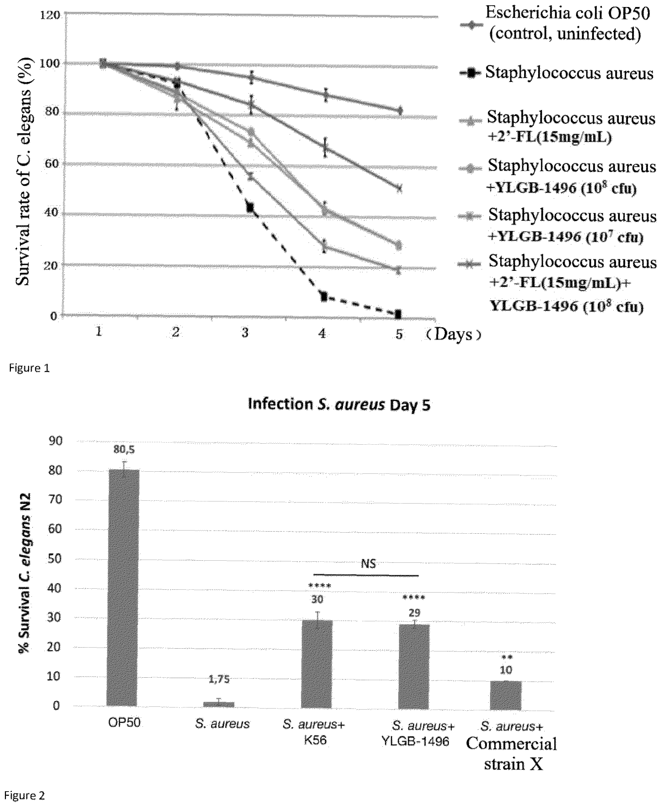

Lifespan is the most direct reflection of changes in *C*. *elegans* before and after exposure, and the experimental results are shown in FIG. 1. After infection with *S*. *aureus,* the survival rate of the *C*. *elegans* was decreased significantly from day 3, and the survival rate of the *C*. *elegans* was only 1.75% on day 5. In contrast, the *C*. *elegans* group treated with 10⁷ CFU of *Bifidobacterium infantis* YLGB-1496 had a 20% survival rate on day 5; the *C*. *elegans* group treated with 10⁸ CFU of *Bifidobacterium infantis* YLGB-1496 had an increased survival rate to 29% on day 5, which was the same as the *C*. *elegans* group treated with 2'-FL and was significantly different from the untreated control group (1.75% survival rate, p < 0.001). In addition, the survival rate of the *C*. *elegans* groups treated with 10⁸ CFU of *Bifidobacterium infantis* YLGB-1496 and human milk oligosaccharide 2'-FL (15 mg/mL) was significantly increased to 51.5%; the survival rate of the *C*. *elegans* group treated with *Lactobacillus paracasei* K56 was 30% on day 5, which was significantly different from the 1.75% survival rate of the control group (*p* < 0.001). It can be seen from FIG. 2 that *Bifidobacterium infantis, Bifidobacterium infantis* compounded with human milk oligosaccharide 2'-FL, *Lactobacillus paracasei* K56, and *Bifidobacterium lactis* commercial strain X can significantly prolong lifespan of *C*. *elegans* (*p* < 0.001), wherein the treatment with *Lactobacillus paracasei* K56 and *Bifidobacterium infantis* YLGB-1496 can increase the survival rate of *C*. *elegans* to about 30%, and the treatment with *Bifidobacterium infantis* YLGB-1496 compounded with human milk oligosaccharide 2'-FL can increase the survival rate of *C*. *elegans* to 51.5%, which is the most effective in significantly prolonging lifespan of the *C*. *elegans* (*p* < 0.001).

The above results show that the *Bifidobacterium longum* subsp. *infantis* strain YLGB-1496 (with the deposit number of CGMCC No. 21109) has good potential in anti-aging and improving innate immunity, and can be used in foods, such as fermented dairy, cheese, dairy-containing beverage, dairy powder or any other food containing the strain and a derivative thereof.

### Example 2: Formula powder containing a composition of added human milk oligosaccharides and Bifidobacterium longum subsp. infantis YLGB-1496

This example provides an infant formula powder, wherein the formula powder has a total protein content of 11.1 g/100 g powder, a fat content of 28.3 g/100 g powder, a carbohydrate content of 52.9 g/100 g powder, and *Bifidobacterium longum* subsp. *infantis* YLGB-1496 content of 1 × 10⁸ CFU/100 g powder.

## Claims

1. Use of a *Bifidobacterium longum* subsp. *infantis* strain in the preparation of a composition for use in anti-aging in individuals, improving innate immunity in individuals, relieving symptoms caused by *Staphylococcus aureus* infection in individuals, and/or inhibiting *Staphylococcus aureus* infection in individuals, wherein the *Bifidobacterium infantis* is *Bifidobacterium infantis* with the deposit number of CGMCC No. 21109.

2. The use according to claim 1, wherein the *Bifidobacterium longum* subsp. *infantis* strain is used in the form of a solid or liquid bacterial preparation for the preparation of the composition.

3. The use according to claim 1, wherein the composition comprises a food composition, a feed composition, or a pharmaceutical composition.

4. The use according to claim 3, wherein the pharmaceutical composition further comprises a pharmaceutically acceptable auxiliary material, and the auxiliary material is an excipient, a diluent, a filler, and/or an absorption enhancer.

5. The use according to claim 3, wherein the pharmaceutical composition is in the form of powder, a lozenge, a granule, a microcapsule, a liquid preparation, and/or other dosage forms.

6. The use according to claim 1, wherein the composition is used to reduce death caused by *Staphylococcus aureus* infection;
preferably, the *Bifidobacterium longum* subsp. *infantis* strain is taken in an amount of 1.0 × 10³ CFU to 1.0 × 10¹² CFU/day, more preferably 1.0 × 10⁷ CFU to 1.0 × 10¹⁰ CFU/day.

7. The use according to claim 1, wherein the composition is used to prolong lifespan;
preferably, the *Bifidobacterium longum subsp. infantis* strain is taken in an amount of 1.0 × 10³ CFU to 1.0 × 10¹² CFU/day, more preferably 1.0 × 10⁷ CFU to 1.0 × 10¹⁰ CFU/day.

8. The use according to claim 1, wherein the composition is used to improve innate immunity;
preferably, the *Bifidobacterium longum subsp. infantis* strain is taken in an amount of 1.0 × 10³ CFU to 1.0 × 10¹² CFU/day, more preferably 1.0 × 10⁷ CFU to 1.0 × 10¹⁰ CFU/day.

9. The use according to any one of claims 6-8, wherein the composition is a food composition.

10. The use according to claim 9, wherein the food is a fermented dairy product, cheese, a dairy beverage, a solid beverage, or milk powder.

11. A composition for use in anti-aging in individuals, improving innate immunity in individuals, relieving symptoms caused by *Staphylococcus aureus* infection in individuals, and/or inhibiting *Staphylococcus aureus* infection in individuals, comprising an effective amount of *Bifidobacterium longum* subsp. *infantis* with the deposit number of CGMCC No. 21109.

12. The composition according to claim 11, wherein the composition comprises a food composition, a feed composition, or a pharmaceutical composition.

13. A method for anti-aging, improving innate immunity in individuals, relieving symptoms caused by *Staphylococcus aureus* infection in individuals, and/or inhibiting *Staphylococcus aureus* infection in individuals, comprising administering to an individual in need thereof an effective amount of *Bifidobacterium longum* subsp. *infantis* with the deposit number of CGMCC No. 21109.

14. The method according to claim 13, wherein the anti-aging is anti-immunosenescence.

15. The method according to claim 13 or 14, wherein the *Bifidobacterium longum* subsp. *infantis* is administered to the individual in an amount of 1.0 × 10³ CFU to 1.0 × 10¹² CFU/day, preferably 1.0 × 10⁷ CFU to 1.0 × 10¹⁰ CFU/day.
